# EUROPÄISCHE PATENTSCHRIFT

(11) **EP 3 457 360 B1**
(45) Veröffentlichungstag und Bekanntmachung des Hinweises auf die Patenterteilung: **30.10.2019**
(21) Anmeldenummer: 17190895.7
(22) Anmeldetag: 13.09.2017
(51) Int. Cl.: G06T 11/00

(54) **REDUZIERUNG VON BEWEGUNGSARTEFAKTEN IN COMPUTERTOMOGRAPHISCHEN BILDDATEN**
REDUCTION OF MOTION ARTEFACTS IN COMPUTER TOMOGRAPHY IMAGE DATA
RÉDUCTION DES ARTEFACTS DE MOUVEMENT DANS LES DONNÉES D'IMAGE TOMOGRAPHIQUES COMMANDÉES PAR ORDINATEUR

(43) Veröffentlichungstag der Anmeldung: 20.03.2019
(73) Patentinhaber: Siemens Healthcare GmbH, 91052 Erlangen (DE)
(72) Erfinder: SCHÖNDUBE, Harald, 91056 Erlangen (DE); KAPPLER, Steffen, 91090 Effeltrich (DE)

(56) Entgegenhaltungen:
- Hanming Zhang ET AL: "Image Prediction for Limited-angle Tomography via Deep Learning with Convolutional Neural Network", , 29. Juli 2016 (2016-07-29), XP055451427, Gefunden im Internet: URL:https://arxiv.org/ftp/arxiv/papers/160 7/1607.08707.pdf [gefunden am 2018-02-14]
- GE WANG: "A Perspective on Deep Imaging", IEEE ACCESS, Bd. 4, 3. November 2016 (2016-11-03), Seiten 8914-8924, XP055451413, USA ISSN: 2169-3536, DOI: 10.1109/ACCESS.2016.2624938
- WÜRFL TOBIAS ET AL: "Deep Learning Computed Tomography", 2. Oktober 2016 (2016-10-02), ECCV 2016 CONFERENCE; [LECTURE NOTES IN COMPUTER SCIENCE; LECT.NOTES COMPUTER], SPRINGER INTERNATIONAL PUBLISHING, CHAM, PAGE(S) 432 - 440, XP047364505, ISSN: 0302-9743 ISBN: 978-3-642-33485-6 [gefunden am 2016-10-02] * das ganze Dokument *

## Beschreibung

Die Erfindung betrifft ein Verfahren zur Bildgebung, eine Bildrekonstruktionseinrichtung, ein Computertomographiesystem, ein Computerprogramm und einen computerlesbaren Datenträger, welche eine Reduktion von Bewegungsartefakten in computertomographischen Bilddaten ermöglichen.

Die Computertomographie ist ein bildgebendes Verfahren, welches vor allem zur medizinischen Diagnostik sowie zur Materialuntersuchung eingesetzt wird. Bei der Computertomographie rotieren zur Aufnahme räumlich dreidimensionaler Bilddaten eine Strahlungsquelle, beispielsweise eine Röntgenquelle, sowie eine mit diesem zusammen wirkende Detektorvorrichtung um ein zu untersuchendes Objekt. Während der Rotationsbewegung werden innerhalb eines Winkelsektors Messdaten aufgenommen. Bei den Projektionsmessdaten handelt es sich um eine Vielzahl von Projektionen, welche Informationen über die Schwächung der Strahlung durch das Untersuchungsobjekt aus verschiedenen Projektionswinkeln enthalten. Aus diesen Projektionen lässt sich ein zweidimensionales Schnittbild oder ein dreidimensionales Volumenbild des Untersuchungsobjektes berechnen. Die Projektionsmessdaten werden auch als Rohdaten bezeichnet bzw. die Projektionsmessdaten können bereits vorverarbeitet sein, so dass beispielsweise detektorbedingte Intensitätsunterschiede der Schwächung reduziert sind. Aus diesen Projektionsmessdaten können dann Bilddaten rekonstruiert werden, beispielsweise mittels der sogenannten gefilterten Rückprojektion oder mittels eines iterativen Rekonstruktionsverfahrens. Bewegt sich das Untersuchungsobjekt beispielsweise während der Aufnahme, so können bei der Rekonstruktion der Bilddaten Unschärfe und Artefakte aufgrund der Bewegung entstehen.

Vielfältige Verfahren zur Abtastung eines Untersuchungsobjektes mit einem Computertomographiesystem sind bekannt. Es werden beispielsweise Kreisabtastungen, sequentielle Kreisabtastungen mit Vorschub oder Spiralabtastungen angewandt. Auch andersartige Abtastungen, die nicht auf Kreisbewegungen beruhen, sind möglich, so z.B. Scans mit linearen Segmenten. Es werden mit Hilfe mindestens einer Röntgenquelle und mindestens einer gegenüberliegenden Detektorvorrichtung Absorptionsdaten des Untersuchungsobjektes aus unterschiedlichen Aufnahmewinkeln aufgenommen und diese so gesammelten Absorptionsdaten bzw. Projektionsmessdaten mittels entsprechender Rekonstruktionsverfahren zu Schnittbildern durch das Untersuchungsobjekt verrechnet.

Zur Rekonstruktion von computertomographischen Bildern aus den Projektionsmessdaten eines Computertomographiesystems, wird heutzutage als Standardverfahren ein so genanntes gefiltertes Rückprojektionsverfahren (Filtered Back Projection; FBP) oder eine iterative Rekonstruktion eingesetzt. Bei gefilterten Rückprojektionsverfahren können aufgrund ihrer approximativen Arbeitsweise Probleme mit so genannten Kegelstrahl-Artefakten, Spiralartefakten und limited-view-Artefakten entstehen. Das gefilterte Rückprojektionsverfahren gehört zur Gruppe der approximativen Rekonstruktionsverfahren. Es existiert ferner die Gruppe der exakten Rekonstruktionsverfahren, welche jedoch derzeit kaum eingesetzt werden. Eine dritte Gruppe von Rekonstruktionsverfahren bilden die iterativen Verfahren.

Mit iterativen Rekonstruktionsverfahren können zumindest manche der oben genannten Limitationen der gefilterten Rückprojektion beseitigt werden. Bei einem solchen iterativen Rekonstruktionsverfahren erfolgt zunächst eine Rekonstruktion von Startbilddaten aus den Projektionsmessdaten. Hierzu kann beispielsweise ein gefiltertes Rückprojektionsverfahren verwendet werden. Das iterative Rekonstruktionsverfahren erzeugt im Anschluss nach und nach verbesserte Bilddaten. Beispielsweise können aus den Startbilddaten mit einem "Projektor", einem Projektionsoperator, welcher das Messsystem mathematisch möglichst gut abbilden sollte, synthetische Projektionsdaten erzeugt werden. Die Differenz zu den Messsignalen wird dann mit dem zu dem Projektor adjungierten Operator rückprojiziert und es wird so ein Residuum-Bild rekonstruiert, mit dem das initiale Bild aktualisiert wird. Die aktualisierten Bilddaten können wiederum verwendet werden, um in einem nächsten Iterationsschritt mit Hilfe des Projektionsoperators neue synthetische Projektionsdaten zu erzeugen, daraus wieder die Differenz zu den Messsignalen zu bilden und ein neues Residuum-Bild zu berechnen, mit dem wieder die Bilddaten der aktuellen Iterationsstufe verbessert werden usw. Beispiele für iterative Rekonstruktionsverfahren sind die algebraische Rekonstruktionstechnik (ART), die simultane algebraische Rekonstruktionstechnik (SART), die iterierte gefilterte Rückprojektion (IFBP), oder auch statistische iterative Bildrekonstruktionstechniken.

Aus der Druckschrift DE 10 2006 007 058 A1 ist ein CT-System mit einer Gantry zur Befestigung von mindestens einem um eine Systemachse drehbaren Fokus/Detektor-System bekannt. Das CT-System besteht aus einem Fokus als Strahlenquelle und einem die vom Fokus ausgehende Strahlung empfangenden Detektor, einem ersten Fokus/Detektor-System und mindestens einem zweiten, drehversetzt zum ersten angeordneten Fokus/Detektor-System, welches sich mechanisch gekoppelt mit dem ersten Fokus/Detektor-System auf der Gantry mitdreht, wobei jedes Fokus/Detektor-System einen Zentralstrahl, der vom Fokus durch die Systemachse zum Detektor reicht, und einen Mittenstrahl, der vom Fokus zur geometrischen Mitte des Detektors reicht, aufweist und mindestens ein Fokus/Detektor-System exzentrisch zur Systemachse angeordnet ist.

Aus der Druckschrift DE 10 2008 051 043 B3 ist ein Röntgen-CT-System und ein Verfahren zur Erstellung tomographischer Aufnahmen mit Hilfe eines Röntgen-CT-Systems mit zwei auf einer Gantry winkelversetzt arbeitenden Strahler-Detektor-Anordnungen mit mindestens zwei unterschiedlichen Röntgenenergiespektren bekannt, wobei mindestens eine erste Aufnahme aus Detektordaten aus zwei Viertelrotationen mit unterschiedlichen Röntgenenergiespektren rekonstruiert und mindestens eine zweite Aufnahme aus Detektordaten einer Abtastung mindestens einer der Strahler-Detektor-Anordnungen über eine Halbrotation erstellt werden. Die Aufnahmen werden einer Hochpassfilterung beziehungsweise einer Tiefpassfilterung bezüglich ihrer Ortsfrequenzen unterzogen und anschließend die gefilterten Aufnahmen zu einer Ergebnisaufnahme kombiniert.

Aus der Druckschrift DE 10 2010 019 016 A1 ist ein Verfahren zur Rekonstruktion von Bilddaten eines bewegten Untersuchungsobjektes aus Messdaten bekannt, wobei die Messdaten zuvor bei einer relativen Rotationsbewegung zwischen einer Strahlungsquelle eines Computertomographiesystems und dem Untersuchungsobjekt erfasst wurden. Aus einem unvollständigen Messdatensatz werden mittels eines iterativen Algorithmus erste Bilddaten rekonstruiert, wobei bei der iterativen Rekonstruktion eine Größe verwendet wird, welche Wahrscheinlichkeits-Informationen betreffend Bildpunktwerte der zu rekonstruierenden Bilddaten enthält.

Mittels zwei um 90° versetzten Strahler-Detektor-Anordnungen können gleichzeitig Daten aufgenommen werden, so dass eine Projektionswinkelabdeckung von 180° bereits nach einer Gantrydrehung um 90° erreicht wird. Dies entspricht einer Verdoppelung der erreichbaren Zeitauflösung. Alternativ kann die Aufnahme in zwei zeitliche Abschnitte über jeweils 90° erfolgen, wobei jeweils derselbe Bewegungszustand des Herzens bei aufeinanderfolgenden Herzschlägen abgebildet wird. Die maximal mögliche Zeitauflösung wird verdoppelt. Außerdem kann eine Erhöhung der Rotationsgeschwindigkeit der Gantry die erreichbare Zeitauflösung erhöhen. Der mechanischen Lösung des Problems der limitierten Zeitauflösung sind jedoch Grenzen gesetzt.

In der Computertomographie ist die Zeitauflösung eines insbesondere einzelnen (Schicht-)Bildes gegeben durch die Zeitspanne, in der die einzelnen Projektionen bzw. die Projektionsmessdaten eines Winkelsektors, aus denen das Bild rekonstruiert wurde, aufgenommen wurden. Für Anwendungen, bei denen die Zeitauflösung kritisch ist, beispielsweise bei der Herzbildgebung, wird deshalb üblicherweise nur die zur Bildrekonstruktion minimal notwendige Anzahl an Projektionen bzw. die Projektionsmessdaten eines minimal notwendigen Winkelsektors verwendet. Im Allgemeinen wird für die bisher gängigen Bildrekonstruktionstechniken von einem minimal erforderlichen Projektionswinkelbereich von 180° ausgegangen. Im Falle einer Rotationsgeschwindigkeit der Gantry, welche das Bildaufnahmesystem bestehend aus Röntgenquelle und Detektorvorrichtung umfasst, von 0,5 Sekunden kann entsprechend des minimal erforderlichen Winkelsektors von 180° eine maximale Zeitauflösung von 0,25 Sekunden erreicht werden, da mindestens die Projektionsdaten von einem halben Umlauf benötigt werden.

Ein Artikel von Hanming Zhang et al.: "Image Prediction for Limitedangle Tomography via Deep Learning with Convolutional Neural Network", 29. Juli 2016, Gefunden im Internet: https://arxiv.org/ftp/arxiv/ papers/1607/1607.08707.pdf offenbart ein Verfahren zur Reduktion von "limited angle"-Artefakten in tomographischen Bildern, welche über einen beschränkten Winkelbereich kleiner als 180 Grad aufgenommen wurden, mittels eines künstlichen neuronalen Netzes.

Wenn sich der darzustellende Untersuchungsbereich des Untersuchungsobjekts während der Aufnahme der Projektionen bzw. Projektionsmessdaten, beispielsweise aus dem minimal erforderlichen Winkelsektor, bewegt, entstehen im rekonstruierten (Ergebnis-)Bild sogenannte Bewegungsartefakte. Die Verminderung bzw. Eliminierung dieser Bewegungsartefakte ist eine Aufgabe der vorliegenden Erfindung.

Es ist Aufgabe der Erfindung, ein Verfahren zur Bildgebung, eine Bildrekonstruktionseinrichtung, ein Computertomographiesystem, ein Computerprogramm und einen computerlesbaren Datenträger, welche eine Reduktion von Bewegungsartefakten bzw. eine Erhöhung der Zeitauflösung ermöglichen.

Die Aufgabe wird erfindungsgemäß gelöst durch ein Verfahren nach Anspruch 1, eine Bildrekonstruktionseinrichtung nach Anspruch 13, ein Computertomographiesystem nach Anspruch 14, ein Computerprogramm nach Anspruch 15 und einen computerlesbaren Datenträger nach Anspruch 16.

Die Erfindung betrifft ein Verfahren zur Bildgebung eines Untersuchungsbereichs eines zu untersuchenden Objekts basierend auf bei einer Rotationsbewegung eines Röntgenquellen-Detektor-Systems um das zu untersuchende Objekt in einem ersten Winkelsektor von mindestens 180° aufgenommenen Projektionsmessdaten. Das Verfahren weist die Schritte des Erzeugens, des Auswählens, des Abgleichens und der Artefaktkorrektur auf. Im Schritt des Erzeugens werden ersten Startbilddaten erzeugt. Im Schritt des Auswählens werden Teilprojektionsmessdaten mit einem zweiten Winkelsektor aus den Projektionsmessdaten ausgewählt, wobei der zweite Winkelsektor ein Teilbereich des ersten Winkelsektors ist. Im Schritt des Abgleichens werden die ersten Startbilddaten oder korrigierte Startbilddaten mit den Teilprojektionsmessdaten abgeglichen, wobei erste Bilddaten erzeugt werden. Im Schritt der Artefaktkorrektur werden die ersten Bilddaten mittels einer trainierten Korrektureinheit korrigiert, wobei die korrigierten Startbilddaten erstellt werden. Die ersten Bilddaten und die korrigierten Startbilddaten weisen jeweils ein im Wesentlichen vollständiges Bild des Untersuchungsbereichs auf.

Die Aufgabe dieser Erfindung ist die Rekonstruktion eines Ergebnisbildes aus computertomographischen Projektionsmessdaten, wobei die zu erreichende Zeitauflösung kleiner ist als die Zeitspanne, innerhalb derer Projektionsdaten in einem ersten Winkelsektor von beispielsweise mindestens 180° gemessen werden. Vorteilhaft können sogenannte "limited angle"-Artefakte vermieden oder reduziert werden, welche beispielsweise bei einer direkten Anwendung von bekannten Rekonstruktionsverfahren, beispielsweise der gefilterten Rückprojektion (engl.: filtered backprojection, Abkürzung: FBP), entstehen. Die ersten Bilddaten und die korrigierten Startbilddaten weisen jeweils ein im Wesentlichen vollständiges Bild des Untersuchungsbereichs auf. Der Untersuchungsbereich kann beispielsweise auf einen Teilbereich eines Schichtbildes bzw. eines Aufnahmebereichs begrenzt sein. Der Untersuchungsbereich kann beispielsweise nur einen begrenzten Teilbereich umfassend einen vorbestimmten anatomischen Teilbereich des zu untersuchenden Objekts bzw. des Untersuchungsbereichs aufweisen. Der Untersuchungsbereich kann beispielsweise im Wesentlichen nur ein einziges Organ, beispielsweise das Herz, und ggf. zusätzlich einen Randbereich des umgebenden Gewebes um das Organ umfassen. Das im Wesentlichen vollständige Bild kann insbesondere gegenüber den ersten Startbilddaten reduzierte "limited angle"-Artefakte bzw. keine "limited angle"-Artefakte aufweisen. Das im Wesentlichen vollständige Bild kann insbesondere gegenüber dem Startbild reduzierte sogenannte direktionale Artefakte bzw. "limited angle"-Artefakte, welche durch die Verwendung des insbesondere weniger als 180° umfassenden zweiten Winkelsektors bedingt sein können, aufweisen. Das im Wesentlichen vollständige Bild kann beispielsweise im Wesentlichen korrekte Bildinformationen des Untersuchungsbereichs aufweisen. In den ersten Startbilddaten können dagegen "limited angle"-Artefakte bzw. direktionale Artefakte umfasst sein. Sogenannte "limited angle"-Artefakte können insbesondere durch Verwendung eines zu kleinen, beispielsweise zweiten, Winkelsektors bei der Rekonstruktion entstehen. Betrachtet man beispielsweise ein Objekt mit einem im Wesentlichen kreisförmigen Querschnitt in der Ebene des Schichtbildes im Untersuchungsbereich, so erhält man durch Verwendung des zweiten Winkelbereichs bei der Rekonstruktion, beispielsweise mittels gefilterter Rückprojektion, in der Regel statt eines kreisförmigen Querschnitts einen zwiebelförmigen oder mandelförmigen Querschnitt, wobei die beiden Spitzen des zwiebelförmigen bzw. mandelförmigen Querschnitts in Richtung der zentralen Projektion innerhalb des zweiten Winkelsektors zeigen. Insbesondere im Differenzbereich des zwiebelförmigen bzw. mandelförmigen Querschnitts und des kreisrunden Querschnitts kann das Bild unvollständig sein, d.h. die Information über das Objekt wird in diesem Differenzbereich nicht vollständig bzw. nicht korrekt wiedergegeben. In dem im Wesentlichen vollständigen Bild kann das Objekt mit einem im Wesentlichen kreisförmigen Querschnitt ebenfalls einen kreisförmigen Querschnitt aufweisen.

Das erfindungsgemäße Verfahren nutzt einen Teilbereich der Projektionsmessdaten, beispielsweise 120°, beispielsweise in Verbindung mit einer Randbedingung, welche auf einer Datenbank basiert, die auf einem Prinzip des dictionary-learnings basieren kann.

Die Projektionsmessdaten können als Sinogramm vorliegen bzw. erzeugt werden, die einen ersten Winkelsektor von mindestens 180° abdecken bzw. umfassen. Es wird insbesondere anschließend ein Teilbereich des Sinogramms ausgewählt, wobei der Teilbereich einen zweiten Winkelsektor umfasst, welcher insbesondere kleiner als der erste Winkelsektor ist und welcher insbesondere weniger als 180° umfasst.

Die ersten Bilddaten bzw. das Ergebnisbild können ausgehend von einem Startbild mittels eines iterativen Verfahrens rekonstruiert werden, in dem sich insbesondere die Schritte des Abgleichens und der Artefaktkorrektur abwechseln. Im Schritt des Abgleichens werden die Startbilddaten mit den Teilprojektionsmessdaten abgeglichen. Das Ergebnis kann als erste Bilddaten gespeichert werden. Im Schritt der Artefaktkorrektur können die ersten Bilddaten beispielsweise mittels einer auf dem Prinzip des dictionary-learning basierenden Datenbank korrigiert werden, wobei die in den ersten Bilddaten vorhandenen "limited angle"-Artefakte sowie eventuelle Bewegungsartefakte, beispielsweise insbesondere im Bereich der Koronararterien, zu entfernen bzw. zu korrigieren. Die Datenbank kann dabei beispielsweise in Form eines elektronischen anatomischen Atlas realisiert sein. Beispielsweise kann ein artefaktbehafteter Bildbereich des ersten Bildes mittels eines in der Datenbank hinterlegten artefaktfreien anatomisch entsprechenden Bildbereichs korrigiert werden. Das Ergebnis wird als korrigierte Startbilddaten gespeichert. Die korrigierten Startbilddaten können anschließend im Schritt des Abgleichens verwendet werden.

Der Schritt des Abgleichens kann insbesondere dazu dienen, die Startbilddaten mit den Teilprojektionsmessdaten vorteilhaft konsistent zu halten. Im Schritt der Artefaktkorrektur kann insbesondere eine Entfernung bzw. eine Verringerung von Artefakten erreicht werden, die aufgrund der Objektbewegung oder der Verwendung des im Vergleich zum ersten Winkelsektor kleineren zweiten Winkelsektors zu erwarten sind.

Das erfindungsgemäße Verfahren kann insbesondere die Verwendung einer auf dem Prinzip des machine-learning basierenden Datenbank in Kombination mit einem iterativen Rekonstruktionsalgorithmus zur Bildrekonstruktion unter Verwendung von Teilprojektionsmessdaten eines zweiten Winkelsektors aufweisend weniger als 180° umfassen. Vorteilhaft kann ein Ergebnisbild bzw. ein Teilbereich eines Untersuchungsbereichs basierend auf Teilprojektionsmessdaten eines zweiten Winkelsektors aufweisend weniger als 180° rekonstruiert werden. Vorteilhaft kann eine erhöhte Zeitauflösung des Ergebnisbildes erreicht werden.

Gemäß einem Aspekt der Erfindung basiert die trainierte Korrektureinheit auf einem maschinellen Lernverfahren, einer statistischen Methode, einer Abbildungsvorschrift, mathematischen Funktionen, einem künstlichen neuronalen Netz oder einer lernenden Datenbank. Gemäß einem Aspekt der Erfindung nutzt die trainierte Korrektureinheit ein maschinelles Lernverfahren, eine statistische Methode, eine Abbildungsvorschrift, mathematischen Funktionen, ein künstliches neuronales Netz oder eine lernende Datenbank. Die lernende Datenbank kann auch als dictionary-learning basierte Datenbank bezeichnet werden. Die trainierte Korrektureinheit kann bevorzugt eine lernende Datenbank nutzen. Vorteilhaft können Muster oder Gesetzmäßigkeiten aus den Trainingsdaten auf die ersten Bilddaten angewendet werden, wobei die korrigierten Startbilddaten erstellt werden. Die Korrektureinheit kann Verknüpfungen oder Gewichtungen von Merkmalen oder Kenngrößen in den Trainingsdaten zur Artefaktkorrektur verwenden.

Unter maschinellen Lernverfahren kann man die künstliche Generierung von Wissen aus Erfahrung bezeichnen. Ein künstliches System lernt aus Beispielen in einer Trainingsphase und kann nach Beendigung der Trainingsphase verallgemeinern. Damit kann die Erkennungseinheit angepasst werden. Die Verwendung maschineller Lernverfahren kann ein Erkennen von Mustern und Gesetzmäßigkeiten in den Trainingsdaten umfassen. Nach der Trainingsphase kann die Erkennungseinheit beispielsweise in bisher unbekannten Bilddaten Merkmale oder Kenngrößen extrahieren. Nach der Trainingsphase kann die Erkennungseinheit beispielsweise basierend auf bisher unbekannten Bilddaten eine Artefaktart erkennen. Vorteilhaft kann aus dem Wissen von bekannten Trainingsdaten ein zuverlässiges Verfahren zur Artefakterkennung abgeleitet werden. Vorteilhaft kann auf der Basis der Erfahrungen der Erkennungseinheit von Mustern oder Gesetzmäßigkeiten eine Erkennung eines Artefakts bzw. einer Artefaktart durchgeführt werden.

Gemäß einem Aspekt der Erfindung werden im Schritt des Erzeugens die Startbilddaten basierend auf den Projektionsmessdaten erzeugt. Die Startbilddaten können auf verschiedene Art und Weisen erzeugt werden. Die Startbilddaten können beispielsweise mittels gefilterter Rückprojektion der Projektionsmessdaten erzeugt werden. Die Startbilddaten können beispielsweise mittels gefilterter Rückprojektion der Teilprojektionsmessdaten mit einer Nebenbedingung erzeugt werden, wobei die Nebenbedingung beispielsweise auf einer Datenbank basieren kann, die auf einem Prinzip des dictionary-learnings basiert. Vorteilhaft kann die Ähnlichkeit der Startbilddaten mit dem Ergebnisbild ausgenutzt werden. Vorteilhaft kann die Anzahl der Iterationsschritte reduziert werden.

Gemäß einem Aspekt der Erfindung werden im Schritt des Erzeugens die Startbilddaten von den Projektionsmessdaten unabhängig erzeugt. Die Startbilddaten können ein leeres Bild oder ein vorbestimmtes Bild umfassen. Das vorbestimmte Bild kann beispielsweise ein künstlich erzeugtes Bild umfassen. Das vorbestimmte Bild kann beispielsweise auf einem bekannten Bild einer Aufnahme eines anderen Untersuchungsobjekts basieren. Vorteilhaft können die Startbilddaten von den Artefakten im Wesentlichen unbeeinflusst sein.

Gemäß einem Aspekt der Erfindung werden die Schritte des Abgleichens und der Artefaktkorrektur iterativ durchgeführt, wobei in einem ersten Durchlauf die ersten Startbilddaten mit den Teilprojektionsmessdaten abgeglichen werden und in mindestens einem weiteren Durchlauf die korrigierten Startbilddaten mit den Teilprojektionsmessdaten abgeglichen werden. Das Verfahren kann bis zu einer Abbruchbedingung iterativ in mehreren Durchläufen durchgeführt werden. Die Abbruchbedingung kann beispielsweise eine Kenngröße des Abgleichens oder eine Kenngröße der Artefaktkorrektur umfassen. Die Kenngröße kann beispielsweise umfassen, dass im Schritt des Abgleichens bzw. der Artefaktkorrektur die Eingangsdaten im Wesentlichen nicht weiter verändert werden. Vorteilhaft kann eine Konvergenz schrittweise in mehreren Durchläufen erreicht werden.

Gemäß einem Aspekt der Erfindung wird der Schritt des Abgleichens im Bildraum durchgeführt. Die ersten Startbilddaten oder die korrigierten Startbilddaten werden mit den Teilprojektionsmessdaten abgeglichen, wobei der Abgleich auf Bilddaten der Teilprojektionsmessdaten basiert. Die Bilddaten der Teilprojektionsmessdaten können mittels gefilterter Rückprojektion erzeugt werden. Das Abgleichen kann im Bildraum durchgeführt werden. Vorteilhaft können wiederholte Vorwärts- und Rückwärtsprojektionen vermieden werden. Vorteilhaft kann das erfindungsgemäße Verfahren beschleunigt werden.

Gemäß einem Aspekt der Erfindung wird der Schritt des Abgleichens im Projektionsdatenraum durchgeführt. Der vor dem Schritt des Abgleichens kann eine Vorwärtsprojektion der korrigierten Startbilddaten durchgeführt werden. Der Schritt des Abgleichens kann eine Rückwärtsprojektion umfassen, sodass die ersten Bilddaten entstehen. Das erfindungsgemäße Verfahren kann einen Wechsel zwischen Bildraum und Projektionsdatenraum umfassen. Vorteilhaft kann eine verbesserte Übereinstimmung des Ergebnisbildes mit den Projektionsmessdaten erreicht werden.

Gemäß einem Aspekt der Erfindung weist das erfindungsgemäße Verfahren ferner einen Schritt des Regularisierens auf.
Die Regularisierung kann eine Modellierung des, insbesondere lokalen, Rauschens im CT-Bild umfassen. Die Regularisierung kann eine Separation von Information und Rauschen auf Basis der statistischen Signifikanz umfassen. Es kann ein vorteilhaftes Kontrast-zu-Rausch-Verhältnis im Ergebnisbild erreicht werden. Die Regularisierung kann die Subtraktion des ermittelten Rauschanteils umfassen. Vorteilhaft kann die Konvergenz der iterativen Artefaktkorrektur gesichert sein. Vorteilhaft kann eine Rauschreduktion oder eine Dosisreduktion erreicht werden.

Gemäß einem Aspekt der Erfindung deckt der zweite Winkelsektor einen Winkel von weniger als 180° ab. Gemäß einem Aspekt der Erfindung deckt der zweite Winkelsektor einen Winkel im Bereich von 100° bis 140° ab. Der zweite Winkelsektor kann bevorzugt 110° bis 130° abdecken. Der zweite Winkelsektor kann besonders bevorzugt etwa 120° abdecken. Vorteilhaft kann eine erhöhte Zeitauflösung erreicht werden.

Gemäß einem Aspekt der Erfindung weist das erfindungsgemäße Verfahren ferner den Schritt des Ausgebens der ersten Bilddaten oder der korrigierten Startbilddaten als Ergebnisbild auf. Als Ergebnisbild können die ersten Bilddaten oder die korrigierten Startbilddaten genutzt werden. Der jeweilige Schritt, bei dem das Ergebnisbild in Form der ersten Bilddaten oder der korrigierten Startbilddaten entsteht, kann für die Erstellung oder Ausgabe des Ergebnisbilds ein größeres Gewicht gegenüber dem anderen Schritt aufweisen. Vorteilhaft kann das Gewicht bzw. der Einfluss des Schritts des Abgleichens oder der Artefaktkorrektur durch Wahl der ersten Bilddaten oder der korrigierten Startbilddaten gewählt werden. Das Ergebnisbild kann an den Nutzer ausgegeben werden oder durch weitere Schritte weiterverarbeitet werden.

Gemäß einem Aspekt der Erfindung wird im Schritt der Artefaktkorrektur ein vorbestimmter anatomischer Teilbereich des zu untersuchenden Objekts in den ersten Bilddaten korrigiert. Das Untersuchungsobjekt kann bevorzugt ein Patient sein. Der vorbestimmte anatomische Teilbereich kann vom Herzen umfasst sein. Der vorbestimmte anatomische Teilbereich kann beispielsweise eine Koronararterie umfassen. Der vorbestimmte Teilbereich kann beispielsweise nur mindestens eine Koronararterie umfassen. Der vorbestimmte anatomische Teilbereich kann insbesondere zur Diagnose nötige Teilbereiche des Untersuchungsbereichs umfassen. Vorteilhaft kann eine Datenbank einen anatomischen Atlas aufweisend den vorbestimmten anatomischen Teilbereichs umfassen. Vorteilhaft kann im vorbestimmten anatomischen Teilbereich eine besonders gute Reduktion der Artefakte erreicht werden.

Die Erfindung betrifft ferner eine Bildrekonstruktionseinrichtung aufweisend Mittel zum Durchführen des erfindungsgemäßen Verfahrens zur Bildgebung. Die Bildrekonstruktionseinrichtung bzw. die Mittel umfassen eine Erzeugungseinheit zum Erzeugen von ersten Startbilddaten, eine Auswahleinheit zum Auswählen von Teilprojektionsmessdaten mit einem zweiten Winkelsektor aus den Projektionsmessdaten, wobei der zweite Winkelsektor ein Teilbereich des ersten Winkelsektors ist, eine Abgleicheinheit zum Abgleichen der ersten Startbilddaten oder von korrigierten Startbilddaten mit den Teilprojektionsmessdaten, wobei erste Bilddaten erzeugt werden, und eine Artefaktkorrektureinheit zur Artefaktkorrektur der ersten Bilddaten mittels einer trainierten Korrektureinheit, wobei die korrigierten Startbilddaten erstellt werden. Vorteilhaft kann das erfindungsgemäße Verfahren in einer Bildrekonstruktionseinrichtung durchgeführt werden. Die Bildrekonstruktionseinrichtung kann von einer Recheneinheit umfasst sein. Die Bildrekonstruktionseinrichtung bzw. die Recheneinheit kann vom Computertomographiesystem umfasst sein. Die Bildrekonstruktionseinrichtung kann beispielsweise Projektionsmessdaten oder Startbilddaten aus einem Cloudspeicher zum Durchführen des Verfahrens verwenden.

Die Erfindung betrifft ferner ein Computertomographiesystem mit einer erfindungsgemäßen Bildrekonstruktionseinrichtung. Vorteilhaft kann das Verfahren zur Bilderzeugung bzw. zur Artefaktkorrektur direkt im Computertomographiesystem durchgeführt werden. Vorteilhaft kann aus den Projektionsmessdaten ein Ergebnisbild erstellt werden und dem Benutzer angezeigt werden.

Die Erfindung betrifft ferner ein Computerprogramm mit Programmcode, um das erfindungsgemäße Verfahren durchzuführen, wenn das Computerprogramm auf einem Computer ausgeführt wird. Die Erfindung betrifft ferner einen computerlesbaren Datenträger mit Programmcode eines Computerprogramms, um das erfindungsgemäße Verfahren durchzuführen, wenn das Computerprogramm auf einem Computer ausgeführt wird.

Die Erfindung betrifft ferner ein Verfahren zum Trainieren der Korrektureinheit aufweisend die Schritte des Erzeugens und des Trainierens. Im Schritt des Erzeugens werden Trainingsdaten erzeugt. Die Trainingsdaten können erste Starbilddaten und Projektionsmessdaten umfassen. Die Trainingsdaten können mittels eines Computertomographiesystems erzeugt werden. Die Trainingsdaten können künstlich, beispielsweise mittels Simulation, erzeugt werden. Das Verfahren zum Trainieren kann einen Schritt des Auswählens aufweisen, wobei Teilprojektionsmessdaten mit einem zweiten Winkelsektor aus den Projektionsmessdaten ausgewählt werden, wobei der zweite Winkelsektor ein Teilbereich des ersten Winkelsektors ist. Das Ergebnisbild kann vorbestimmt sein. Das vorbestimmte Ergebnisbild kann ein artefaktreduziertes oder ein artefaktfreies Ergebnisbild sein. Das Verfahren zum Trainieren kann ferner einen Schritt des Abgleichens aufweisen, wobei die ersten Startbilddaten oder korrigierte Startbilddaten mit den Teilprojektionsmessdaten abgeglichen werden, wobei erste Bilddaten erzeugt werden. Das Verfahren zum Trainieren kann ferner einen Schritt der Artefaktkorrektur aufweisen, wobei die ersten Bilddaten mittels einer trainierten Korrektureinheit korrigiert werden, wobei die korrigierten Startbilddaten erstellt werden. Die ersten Bilddaten und die korrigierten Startbilddaten können jeweils ein vollständiges Bild des Untersuchungsbereichs aufweisen.

Im Schritt des Trainierens wird die Erkennungseinheit basierend auf den Trainingsdaten trainiert. Im Schritt des Trainierens bzw. Anpassens kann die Korrektureinheit basierend auf den Trainingsdaten angepasst werden. Der Schritt des Trainierens kann insbesondere ein maschinelles Lernverfahren umfassen, wobei auch eine statistische Methode, eine Abbildungsvorschrift oder ein künstlichen neuronalen Netz umfasst sein können. Die statistische Methode kann beispielsweise Fuzzylogik, eine selbstorganisierende Karte, Stichprobenwiederholung (engl.: Resampling), Mustererkennung oder Support Vector Machine umfassen. Das maschinelle Lernverfahren kann Aspekte von Data-Mining umfassen. Das maschinelle Lernverfahren kann ein symbolisches System oder ein subsymbolisches System, beispielsweise ein künstliches neuronales Netz mit oder ohne Regression, umfassen. Das maschinelle Lernen kann ein überwachtes, teilüberwachtes, unüberwachtes, bestärkendes oder aktives Lernen umfassen. Das maschinelle Lernverfahren kann Batch-Lernen, bei dem alle Trainingsdaten gleichzeitig vorhanden sind und beispielsweise nach Bearbeiten aller Trainingsdaten Muster und Gesetzmäßigkeiten von der Korrektureinheit erlernt werden, umfassen. Das maschinelle Lernen kann ein kontinuierliches, inkrementelles oder sequentielles Lernverfahren umfassen, wobei die Muster und Gesetzmäßigkeiten zeitlich versetzt entwickelt werden. Beim kontinuierlichen, inkrementellen oder sequentiellen Lernverfahren können die Trainingsdaten nach einmaligem Ausführen und beispielsweise Gewichte anpassen verloren gehen. Beim Batch-Lernen oder beim kontinuierlichen, inkrementellen oder sequentiellen Lernverfahren können die Trainingsdaten gespeichert vorliegen und die Trainingsdaten können wiederholbar zugreifbar sein. Das maschinelle Lernverfahren kann beispielsweise Deep-Learning-Methoden oder Shallow-Learning-Methoden umfassen. Vorteilhaft können die Kenntnisse aus bekannten Trainingsdaten auf unbekannte Teilprojektionsmessdaten angewendet werden. Vorteilhaft kann die Korrektureinheit durch das Trainieren eine zuverlässige Artefaktkorrektur ermöglichen. Projektionsmessdaten aus dem Verfahren zur Bildgebung können zusätzlich zum Trainieren der Korrektureinheit verwendet werden, beispielsweise um statistische Wahrscheinlichkeiten des Auftretens von Merkmalen oder Kenngrößen durch eine zunehmend größere Datenbasis zu verbessern. Das Verfahren des Trainierens kann mittels einer Trainingseinheit durchgeführt werden. Die Trainingseinheit kann von einer Recheneinheit umfasst sein.

Nachfolgend werden Ausführungsbeispiele der Erfindung anhand von Zeichnungen näher erläutert. Hierbei zeigt:
FIG 1 schematisch eine Darstellung des erfindungsgemäßen Verfahrens zur Bildgebung gemäß einer ersten Ausführungsform;
FIG 2 schematisch eine Darstellung des erfindungsgemäßen Verfahrens zur Bildgebung gemäß einer zweiten Ausführungsform;
FIG 3 schematisch eine Darstellung des erfindungsgemäßen Verfahrens zur Bildgebung gemäß einer dritten Ausführungsform;
FIG 4 schematisch eine Darstellung des erfindungsgemäßen Verfahrens zum Trainieren der Korrektureinheit; und
FIG 5 schematisch ein Konzept eines erfindungsgemäßen Computertomographiesystems.

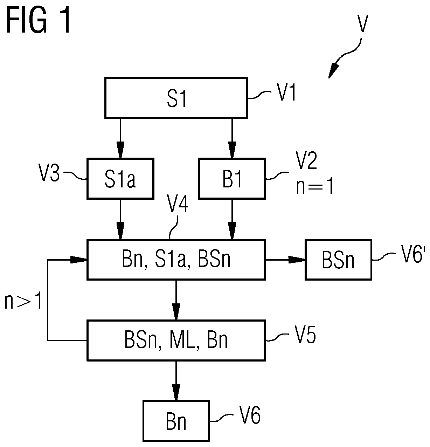

Die Fig. 1 zeigt eine beispielhafte Ausführungsform des erfindungsgemäßen Verfahrens V zur Bildgebung gemäß einer ersten Ausführungsform. Das Verfahren V zur Bildgebung eines Untersuchungsbereichs eines zu untersuchenden Objekts basiert auf bei einer Rotationsbewegung eines Röntgenquellen-Detektor-Systems um das zu untersuchende Objekt in einem ersten Winkelsektor von mindestens 180° aufgenommenen Projektionsmessdaten S1. Die Projektionsmessdaten S1 können in einem ersten Schritt des Aufnehmens V1 aufgenommen werden. Das Verfahren V weist die Schritte des Erzeugens V2, des Auswählens V3, des Abgleichens V4 und der Artefaktkorrektur V5 auf. Im Schritt des Erzeugens V2 werden erste Startbilddaten B1 erzeugt. Im Schritt des Auswählens V3 werden Teilprojektionsmessdaten Sla mit einem zweiten Winkelsektor aus den Projektionsmessdaten S1 ausgewählt, wobei der zweite Winkelsektor ein Teilbereich des ersten Winkelsektors ist. Der zweite Winkelsektor deckt einen Winkel von weniger als 180° ab. Der zweite Winkelsektor deckt bevorzugt einen Winkel im Bereich von 100° bis 140°, insbesondere bevorzugt von 120°, ab. Im Schritt des Abgleichens V4 werden die ersten Startbilddaten B1 oder die korrigierten Startbilddaten Bn mit den Teilprojektionsmessdaten Sla abgeglichen, wobei erste Bilddaten BSn erzeugt werden. Im Schritt der Artefaktkorrektur V5 werden die ersten Bilddaten BSn mittels einer trainierten Korrektureinheit ML korrigiert, wobei die (neuen) korrigierten Startbilddaten Bn erstellt werden. Die ersten Bilddaten BSn und die korrigierten Startbilddaten Bn weisen jeweils ein im Wesentlichen vollständiges Bild des Untersuchungsbereichs auf. Im Schritt des Ausgebens V6, V6' können die ersten Bilddaten BSn oder die korrigierten Startbilddaten Bn als Ergebnisbild ausgegeben werden. Die trainierte Korrektureinheit ML basiert auf einem maschinellen Lernverfahren, einer statistischen Methode, einer Abbildungsvorschrift, mathematischen Funktionen, einem künstlichen neuronalen Netz oder einer lernenden Datenbank. Im Schritt des Erzeugens V2 werden die Startbilddaten B1 basierend auf den Projektionsmessdaten S1 erzeugt. Alternativ können im Schritt des Erzeugens V2 die Startbilddaten B1 von den Projektionsmessdaten S1 unabhängig erzeugt werden. Die Schritte des Abgleichens V4 und der Artefaktkorrektur V5 werden iterativ durchgeführt, wobei in einem ersten Durchlauf (n=1) die ersten Startbilddaten B1 mit den Teilprojektionsmessdaten Sla abgeglichen werden und in mindestens einem weiteren Durchlauf (n>1) die korrigierten Startbilddaten Bn mit den Teilprojektionsmessdaten Sla abgeglichen werden. Im Schritt der Artefaktkorrektur V5 kann bevorzugt ein vorbestimmter anatomischer Teilbereich des zu untersuchenden Objekts in den ersten Bilddaten BSn korrigiert werden.

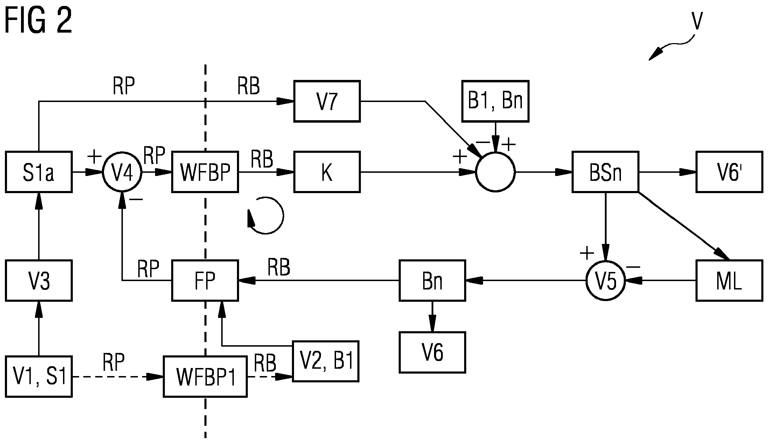

Die Fig. 2 zeigt eine beispielhafte Ausführungsform des erfindungsgemäßen Verfahrens zur Bildgebung gemäß einer zweiten Ausführungsform. Im Schritt des Erzeugens V2 werden erste Startbilddaten B1 basierend auf den Projektionsmessdaten S1 mittels einer gewichteten gefilterten Rückprojektion WFBP1 erzeugt. Es findet ein Übergang vom Projektionsdatenraum RP zum Bildraum RB statt. In der Darstellung trennt die gestrichelte Linie den Projektionsdatenraum RP und den Bildraum RB. Alternativ können im Schritt des Erzeugens V2 die Startbilddaten B1 von den Projektionsmessdaten S1 unabhängig erzeugt werden. Im ersten Durchlauf werden die ersten Startbilddaten B1 mit den Teilprojektionsmessdaten Sla abgeglichen. Die Startbilddaten B1 können dabei mittels einer Vorwärtsprojektion FP in den Projektionsdatenraum RP zurücküberführt werden, sodass der Schritt des Abgleichens V4 im Projektionsdatenraum durchgeführt wird. Es können Korrekturbilddaten K erzeugt werden, indem ein Differenzdatensatz aus den Teilprojektionsmessdaten Sla und der Vorwärtsprojektion FP der Startbilddaten B1 gebildet wird. Aus dem Differenzdatensatz kann mittels gewichteter gefilterter Rückprojektion WFBP ein Korrekturbilddaten K erstellt werden. Die Korrekturbilddaten K können im Bildraum RB verwendet werden, um durch Addition der Korrekturbilddaten K und der Startbilddaten B1 erste Bilddaten BSn zu erzeugen. Im Schritt der Artefaktkorrektur V5 werden die ersten Bilddaten BSn mittels einer trainierten Korrektureinheit ML korrigiert, wobei die korrigierten Startbilddaten Bn erstellt werden. Als Eingangsgröße erhält die trainierte Korrektureinheit ML die ersten Bilddaten BSn. Basierend auf den ersten Bilddaten BSn bestimmt die trainierte Korrektureinheit ML ein Artefaktkorrekturanteil. Der Artefaktkorrekturanteil und die ersten Bilddaten BSn werden addiert, dadurch entstehen korrigierte Startbilddaten Bn.

In den weiteren Durchläufen werden die korrigierten Startbilddaten Bn mittels einer Vorwärtsprojektion FP in den Projektionsdatenraum RP zurücküberführt, sodass der Schritt des Abgleichens V4 im Projektionsdatenraum RP durchgeführt wird. In den weiteren Durchläufen werden die korrigierten Startbilddaten Bn mit den Teilprojektionsmessdaten Sla abgeglichen. Die korrigierten Startbilddaten Bn können dabei mittels einer Vorwärtsprojektion FP in den Projektionsdatenraum RP zurücküberführt werden, sodass der Schritt des Abgleichens V4 im Projektionsdatenraum RP durchgeführt wird. Es können Korrekturbilddaten K erzeugt werden, indem ein Differenzdatensatz aus den Teilprojektionsmessdaten Sla und der Vorwärtsprojektion FP der korrigierten Startbilddaten Bn gebildet wird. Aus dem Differenzdatensatz können mittels gewichteter gefilterter Rückprojektion WFBP Korrekturbilddaten K erstellt werden. Das Korrekturbilddaten K können im Bildraum RB verwendet werden, um durch Addition der Korrekturbilddaten K und der korrigierten Startbilddaten Bn erste Bilddaten BSn zu erzeugen.

Das Verfahren kann ferner einen Schritt des Regularisierens V7 umfassen, wobei das Regularisieren V7 auf den Teilprojektionsmessdaten Sla basiert und bei der Summenbildung der Korrekturbilddaten K und der Startbilddaten B1 bzw. der korrigierten Startbilddaten Bn berücksichtigt wird.

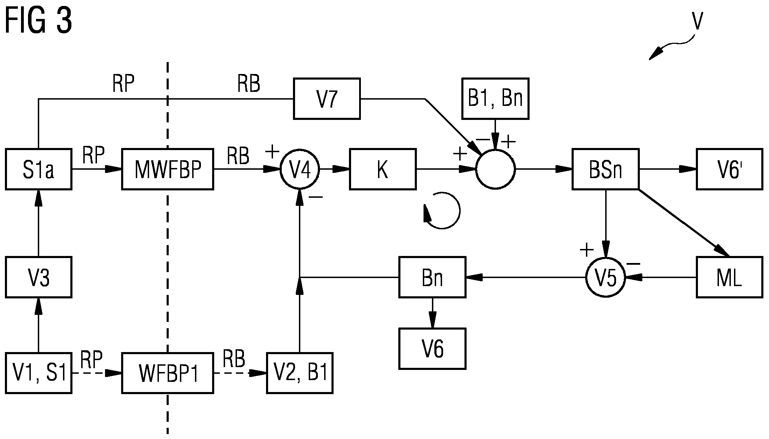

Die Fig. 3 zeigt eine beispielhafte Ausführungsform des erfindungsgemäßen Verfahrens zur Bildgebung gemäß einer dritten Ausführungsform. Im Schritt des Erzeugens V2 werden erste Startbilddaten B1 basierend auf den Projektionsmessdaten S1 mittels einer gewichteten gefilterten Rückprojektion WFBP1 erzeugt. Alternativ können im Schritt des Erzeugens V2 die Startbilddaten B1 von den Projektionsmessdaten S1 unabhängig erzeugt werden. Im ersten Durchlauf werden die ersten Startbilddaten B1 mit den Teilprojektionsmessdaten Sla abgeglichen, wobei Teilprojektionsmessdaten Sla mittels einer einmaligen gewichteten gefilterten Rückprojektion MWFBP in den Bildraum RB überführt werden, sodass der Schritt des Abgleichens V4 im Bildraum RB durchgeführt wird. Es können Korrekturbilddaten K erzeugt werden, indem ein Differenzdatensatz aus der einmaligen gewichteten gefilterten Rückprojektion der Teilprojektionsmessdaten Sla und den Startbilddaten B1 gebildet wird. Der Differenzdatensatz entspricht den Korrekturbilddaten K. Die Korrekturbilddaten K können verwendet werden, um durch Addition der Korrekturbilddaten K und der Startbilddaten B1 erste Bilddaten BSn zu erzeugen. Im Schritt der Artefaktkorrektur V5 werden die ersten Bilddaten BSn mittels einer trainierten Korrektureinheit ML korrigiert, wobei die korrigierten Startbilddaten Bn erstellt werden. Als Eingangsgröße erhält die trainierte Korrektureinheit ML die ersten Bilddaten BSn. Basierend auf den ersten Bilddaten BSn bestimmt die trainierte Korrektureinheit ML ein Artefaktkorrekturanteil. Der Artefaktkorrekturanteil und die ersten Bilddaten BSn werden addiert, dadurch entstehen korrigierte Startbilddaten Bn.

In den weiteren Durchläufen werden die korrigierten Startbilddaten Bn mit den Teilprojektionsmessdaten Sla abgeglichen, wobei die einmalige gewichtete gefilterte Rückprojektion MWFBP der Teilprojektionsmessdaten Sla in den weiteren Durchläufen erneut verwendet wird ohne eine erneute gewichtete gefilterte Rückprojektion der Teilprojektionsmessdaten Sla durchzuführen. Es können Korrekturbilddaten K erzeugt werden, indem ein Differenzdatensatz aus der einmaligen gewichteten gefilterten Rückprojektion der Teilprojektionsmessdaten Sla und den korrigierten Startbilddaten Bn gebildet wird. Der Differenzdatensatz entspricht den Korrekturbilddaten K. Die Korrekturbilddaten K werden verwendet, um durch Addition der Korrekturbilddaten K und der korrigierten Startbilddaten Bn erste Bilddaten BSn zu erzeugen.

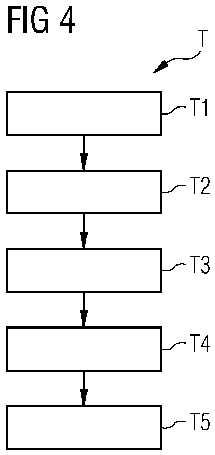

Die Fig. 4 zeigt eine beispielhafte Ausführungsform des erfindungsgemäßen Verfahrens zum Trainieren der Korrektureinheit. Die Erfindung betrifft ferner ein Verfahren T zum Trainieren der Korrektureinheit aufweisend die Schritte des Erzeugens T1 und des Trainierens T5. Im Schritt des Erzeugens T1 werden Trainingsdaten erzeugt. Die Trainingsdaten umfassen beispielsweise erste Starbilddaten und Projektionsmessdaten. Die Trainingsdaten können mittels eines Computertomographiesystems erzeugt werden. Die Trainingsdaten können alternativ künstlich, beispielsweise mittels Simulation, erzeugt werden. Das Verfahren zum Trainieren T kann einen Schritt des Auswählens T2 aufweisen, wobei Teilprojektionsmessdaten mit einem zweiten Winkelsektor aus den Projektionsmessdaten ausgewählt werden, wobei der zweite Winkelsektor ein Teilbereich des ersten Winkelsektors ist. Das Ergebnisbild kann vorbestimmt sein. Das vorbestimmte Ergebnisbild kann ein artefaktreduziertes oder ein artefaktfreies Ergebnisbild sein. Das Verfahren zum Trainieren T kann ferner einen Schritt des Abgleichens T3 aufweisen, wobei die ersten Startbilddaten oder korrigierte Startbilddaten mit den Teilprojektionsmessdaten abgeglichen werden, wobei erste Bilddaten erzeugt werden. Das Verfahren zum Trainieren T kann ferner einen Schritt der Artefaktkorrektur T4 aufweisen, wobei die ersten Bilddaten mittels einer trainierten Korrektureinheit korrigiert werden, wobei die korrigierten Startbilddaten erstellt werden. Die ersten Bilddaten und die korrigierten Startbilddaten können jeweils ein vollständiges Bild des Untersuchungsbereichs aufweisen. Im Schritt des Trainierens T5 wird die Erkennungseinheit basierend auf den Trainingsdaten trainiert. Im Schritt des Trainierens T5 bzw. Anpassens kann die Korrektureinheit basierend auf den Trainingsdaten angepasst werden. Der Schritt des Trainierens T5 kann insbesondere ein maschinelles Lernverfahren umfassen, wobei auch eine statistische Methode, eine Abbildungsvorschrift oder ein künstlichen neuronalen Netz umfasst sein können.

Die statistische Methode umfasst beispielsweise Fuzzylogik, eine selbstorganisierende Karte, Stichprobenwiederholung (engl.: Resampling), Mustererkennung oder Support Vector Machine. Das maschinelle Lernverfahren kann Aspekte von Data-Mining umfassen. Das maschinelle Lernverfahren kann ein symbolisches System oder ein subsymbolisches System, beispielsweise ein künstliches neuronales Netz mit oder ohne Regression, umfassen. Das maschinelle Lernen kann ein überwachtes, teilüberwachtes, unüberwachtes, bestärkendes oder aktives Lernen umfassen. Das maschinelle Lernverfahren kann Batch-Lernen, bei dem alle Trainingsdaten gleichzeitig vorhanden sind und beispielsweise nach Bearbeiten aller Trainingsdaten Muster und Gesetzmäßigkeiten von der Korrektureinheit erlernt werden, umfassen. Das maschinelle Lernen kann ein kontinuierliches, inkrementelles oder sequentielles Lernverfahren umfassen, wobei die Muster und Gesetzmäßigkeiten zeitlich versetzt entwickelt werden. Beim kontinuierlichen, inkrementellen oder sequentiellen Lernverfahren können die Trainingsdaten nach einmaligem Ausführen und beispielsweise Gewichte anpassen verloren gehen. Beim Batch-Lernen oder beim kontinuierlichen, inkrementellen oder sequentiellen Lernverfahren können die Trainingsdaten gespeichert vorliegen und die Trainingsdaten können wiederholbar zugreifbar sein. Das maschinelle Lernverfahren kann beispielsweise Deep-Learning-Methoden oder Shallow-Learning-Methoden umfassen. Das Verfahren des Trainierens wird mittels einer Trainingseinheit durchgeführt. Die Trainingseinheit ist beispielsweise von einer Recheneinheit umfasst.

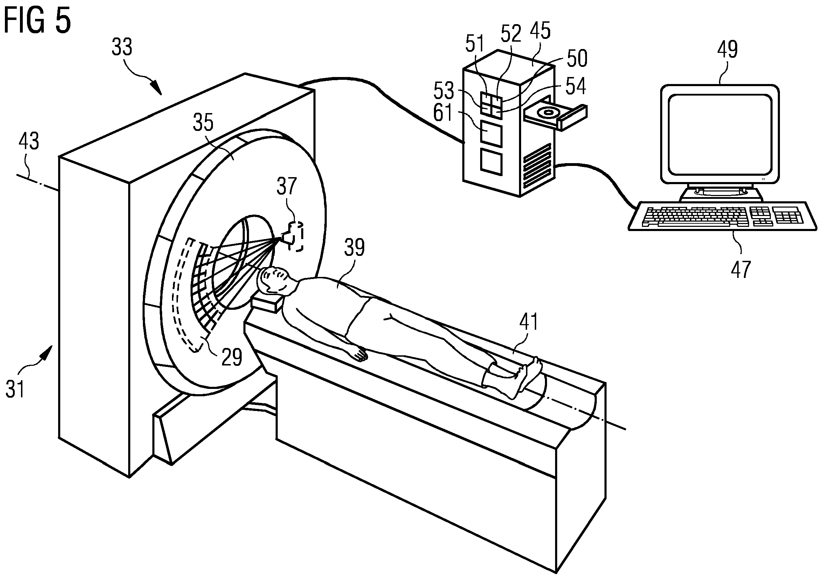

Die Fig. 5 zeigt eine beispielhafte Ausführungsform des erfindungsgemäßen Computertomographiesystems 31 zum Ausführen des erfindungsgemäßen Verfahrens zur Bildgebung. Das Computertomographiesystem 31 beinhaltet eine Gantry 33 mit einem Rotor 35. Der Rotor 35 umfasst eine Röntgenquelle 37 und die Detektorvorrichtung 29. Das Untersuchungsobjekt 39 ist auf der Patientenliege 41 gelagert und ist entlang der Rotationsachse z 43 durch die Gantry 33 bewegbar. Das Computertomographiesystem 31 umfasst ferner eine Recheneinheit 45. Zur Steuerung und Berechnung der Schnittbilder sowie zur Durchführung des erfindungsgemäßen Verfahrens insbesondere zur Bildgebung wird eine Bildrekonstruktionseinrichtung 50 verwendet. Die Bildrekonstruktionseinheit 50 kann eine Erzeugungseinheit 51, eine Auswahleinheit 52, eine Abgleicheinheit 53 und eine Artefaktkorrektureinheit 54 umfassen. Die Bildrekonstruktionseinrichtung 50 kann von der Recheneinheit 45 umfasst sein. Eine Eingabeeinrichtung 47 und eine Ausgabevorrichtung 49 sind mit der Rechnereinheit 45 verbunden. Die Recheneinheit 45 kann ferner die Trainingseinheit 61 umfassen. Ein computerlesbarer Datenträger mit Programmcode eines Computerprogramms kann von der Recheneinheit 45 eingelesen werden bzw. von der Recheneinheit 45 umfasst sein, um das Verfahren zur Bildgebung, wenn das Computerprogramm auf einem Computer bzw. der Recheneinheit 45 ausgeführt wird, durchzuführen.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung, wie durch die beigefügten Ansprüche definiert, zu verlassen.

## Patentansprüche

1. Verfahren (V) zur Bildgebung eines Untersuchungsbereichs eines zu untersuchenden Objekts (39) basierend auf bei einer Rotationsbewegung eines Röntgenquellen-Detektor-Systems (29,37) um das zu untersuchende Objekt (39) in einem ersten Winkelsektor von mindestens 180° aufgenommenen Projektionsmessdaten (S1), aufweisend die Schritte:
- Erzeugen (V2) von ersten Startbilddaten (B1),
- Auswählen (V3) von Teilprojektionsmessdaten (Sla) mit einem zweiten Winkelsektor aus den Projektionsmessdaten (S1), wobei der zweite Winkelsektor ein Teilbereich des ersten Winkelsektors ist,
- Abgleichen (V4) der ersten Startbilddaten (B1) oder von korrigierten Startbilddaten (Bn) mit den Teilprojektionsmessdaten (S1a), wobei erste Bilddaten (BSn) erzeugt werden,
- Artefaktkorrektur (V5) der ersten Bilddaten (BSn) mittels einer trainierten Korrektureinheit (ML), wobei die korrigierten Startbilddaten (Bn) erstellt werden, und
wobei die ersten Bilddaten (BSn) und die korrigierten Startbilddaten (Bn) jeweils ein im Wesentlichen vollständiges Bild des Untersuchungsbereichs aufweisen.

2. Verfahren (V) nach Anspruch 1, wobei die trainierte Korrektureinheit auf einem maschinellen Lernverfahren, einer statistischen Methode, einer Abbildungsvorschrift, mathematischen Funktionen, einem künstlichen neuronalen Netz oder einer lernenden Datenbank basiert.

3. Verfahren (V) nach einem der vorangehenden Ansprüche, wobei im Schritt des Erzeugens (V2) die Startbilddaten (B1) basierend auf den Projektionsmessdaten (S1) erzeugt werden.

4. Verfahren (V) nach einem der Ansprüche 1 bis 2, wobei im Schritt des Erzeugens (V2) die Startbilddaten (B1) von den Projektionsmessdaten S1 unabhängig erzeugt werden.

5. Verfahren nach einem der vorangehenden Ansprüche, wobei die Schritte des Abgleichens und der Artefaktkorrektur iterativ durchgeführt werden, wobei in einem ersten Durchlauf die ersten Startbilddaten (B1) mit den Teilprojektionsmessdaten (Sla) abgeglichen werden und in mindestens einem weiteren Durchlauf die korrigierten Startbilddaten (Bn) mit den Teilprojektionsmessdaten (Sla) abgeglichen werden.

6. Verfahren nach einem der vorangehenden Ansprüche, wobei der Schritt des Abgleichens (V4) im Bildraum (RB) durchgeführt wird.

7. Verfahren nach einem der Ansprüche 1 bis 5, wobei der Schritt des Abgleichens (V4) im Projektionsdatenraum (RP) durchgeführt wird.

8. Verfahren nach einem der vorangehenden Ansprüche, ferner aufweisend einen Schritt des Regularisierens.

9. Verfahren (V) nach einem der vorangehenden Ansprüche, wobei der zweite Winkelsektor einen Winkel von weniger als 180° abdeckt.

10. Verfahren (V) nach Anspruch 9, wobei der zweite Winkelsektor einen Winkel im Bereich von 100° bis 140° abdeckt.

11. Verfahren nach einem der vorangehenden Ansprüche, ferner aufweisend den Schritt des Ausgebens (V6, V6') der ersten Bilddaten (BSn) oder der korrigierten Startbilddaten (Bn) als Ergebnisbild.

12. Verfahren nach einem der vorangehenden Ansprüche, wobei im Schritt der Artefaktkorrektur (V5) ein vorbestimmter anatomischer Teilbereich des zu untersuchenden Objekts (39) in den ersten Bilddaten (BSn) korrigiert wird.

13. Bildrekonstruktionseinrichtung (50) aufweisend Mittel zum Durchführen eines Verfahrens (V) gemäß einem der Ansprüche 1 bis 12, aufweisend:
- eine Erzeugungseinheit (51) zum Erzeugen (V2) von ersten Startbilddaten (B1),
- eine Auswahleinheit (52) zum Auswählen (V3) von Teilprojektionsmessdaten (Sla) mit einem zweiten Winkelsektor aus den Projektionsmessdaten (S1), wobei der zweite Winkelsektor ein Teilbereich des ersten Winkelsektors ist,
- eine Abgleicheinheit (53) zum Abgleichen (V4) der ersten Startbilddaten (B1) oder von korrigierten Startbilddaten (Bn) mit den Teilprojektionsmessdaten (S1a), wobei erste Bilddaten (BSn) erzeugt werden, und
- eine Artefaktkorrektureinheit (54) zur Artefaktkorrektur (V5) der ersten Bilddaten (BSn) mittels einer trainierten Korrektureinheit (ML), wobei die korrigierten Startbilddaten (Bn) erstellt werden.

14. Computertomographiesystem mit einer Bildrekonstruktionseinrichtung (50) nach Anspruch 13.

15. Computerprogramm mit Programmcode, um das Verfahren (V) nach einem der Ansprüche 1 bis 12 durchzuführen, wenn das Computerprogramm auf einem Computer ausgeführt wird.

16. Computerlesbarer Datenträger mit Programmcode eines Computerprogramms, um das Verfahren (V) nach einem der Ansprüche 1 bis 12 durchzuführen, wenn das Computerprogramm auf einem Computer ausgeführt wird.

## Claims

1. Method (V) for imaging a region of interest of an object (39) to be examined based on projection measurement data (S1) recorded during a rotational movement of an X-ray source-detector system (29,37) around the object to be examined (39) in a first angular sector of at least 180° comprising the steps:
- generation (V2) of first start-image data (B1),
- selection (V3) of partial projection measurement data (Sla) with a second angular sector from the projection measurement data (S1), wherein the second angular sector is a subregion of the first angular sector,
- comparison (V4) of the first start-image data (B1) or corrected start-image data (Bn) with the partial projection measurement data (S1a), wherein first image data (BSn) is generated,
- artefact correction (V5) of the first image data (BSn) by means of a trained correction unit (ML), wherein the corrected start-image data (Bn) is created, and
wherein the first image data (BSn) and the corrected start-image data (Bn) in each case comprise a substantially complete image of the region of interest.

2. Method (V) according to claim 1, wherein the trained correction unit is based on a machine learning method, a statistical method, a mapping protocol, mathematical functions, an artificial neural network or a learning database.

3. Method (V) according to one of the preceding claims, wherein, in the generation step (V2), the start-image data (B1) is generated based on the projection measurement data (S1).

4. Method (V) according to one of claims 1 to 2, wherein in the generation step (V2), the start-image data (B1) is generated independently of the projection measurement data S1.

5. Method according to one of the preceding, wherein the comparison and artefact correction steps are performed iteratively, wherein, in a first pass, the first start-image data (B1) is compared with the partial projection measurement data (Sla) and, in at least one further pass, the corrected start-image data (Bn) is compared with the partial projection measurement data (S1a).

6. Method according to one of the preceding claims, wherein the comparison step (V4) is performed in the image space (RB) .

7. Method according to one of claims 1 to 5, wherein the comparison step (V4) is performed in the projection data space (RP).

8. Method according to one of the preceding claims, further comprising a regularisation step.

9. Method (V) according to one of the preceding claims,
wherein the second angular sector covers an angle of less than 180°.

10. Method (V) according to claim 9, wherein the second angular sector covers an angle in the range of 100° to 140°.

11. Method according to one of the preceding claims, further comprising the step of outputting (V6, V6') the first image data (BSn) or the corrected start-image data (Bn) as a result image.

12. Method according to one of the preceding claims, wherein, in the artefact correction step (V5), a predetermined anatomical subregion of the object (39) to be examined is corrected in the first image data (BSn).

13. Image reconstruction device (50) comprising means for performing a method (V) according to one of claims 1 to 12 comprising:
- a generating unit (51) for generating (V2) first start-image data (B1),
- a selecting unit (52) for selecting (V3) partial projection measurement data (Sla) with a second angular sector from the projection measurement data (S1), wherein the second angular sector is a subregion of the first angular sector,
- a comparing unit (53) for comparing (V4) the first start-image data (B1) or corrected start-image data (Bn) with the partial projection measurement data (S1a), wherein first image data (BSn) is generated, and
- an artefact correction unit (54) for artefact correction (V5) of the first image data (BSn) by means of a trained correction unit (ML), wherein the corrected start-image data (Bn) is created.

14. Computed tomography system with an image reconstruction device (50) according to claim 13.

15. Computer program with program code for performing the method (V) according to one of claims 1 to 12 when the computer program is executed on a computer.

16. Computer-readable data medium with program code of a computer program for performing the method (V) according to one of claims 1 to 12, when the computer program is executed on a computer.

## Revendications

1. Procédé (V) d'imagerie d'une partie à examiner d'un objet (39) à examiner, reposant sur des données (S1) de mesure de projection enregistrées lors d'un mouvement de rotation d'un système (29, 37) source-détecteur de rayon X autour de l'objet (39) à examiner dans un premier secteur angulaire d'au moins 180°, comprenant les stades :
- production (V2) de premières données (B1) d'image initiales,
- sélection (V3) de données (S1a) de mesure de projection partielles avec un deuxième secteur angulaire dans les données (S1) de mesure de projection, le deuxième secteur angulaire étant une partie du premier secteur angulaire,
- égalisation (V4) des premières données (B1) d'image initiales ou de données (Bn) d'image initiales corrigées avec les données (S1a) de mesure de projection partielles, des premières données (BSn) d'image étant produites,
- correction (V5) d'artefacts des premières données (BSn) d'image au moyen d'une unité (ML) de correction ayant subi un apprentissage, les données (Bn) d'image initiales corrigées étant produites et
dans lequel les premières données (BSn) d'image et les données (Bn) d'image initiales corrigées ont, respectivement, une image sensiblement complète de la partie à examiner.

2. Procédé (V) suivant la revendication 1, dans lequel l'unité de correction ayant subi un apprentissage repose sur un procédé d'apprentissage automatique, une méthode statistique, une prescription de reproduction, des fonctions mathématiques, un réseau neuronal artificiel ou une base de données d'apprentissage.

3. Procédé (V) suivant l'une des revendications précédentes, dans lequel, dans le stade de la production (V2), on produit les données (B1) d'image initiales sur la base des données (S1) de mesure de projection.

4. Procédé (V) suivant l'une des revendications 1 à 2, dans lequel, dans le stade de la production (V2), on produit les données (B1) d'image initiales indépendamment des données (S1) de mesure de projection.

5. Procédé suivant l'une des revendications précédentes, dans lequel on effectue, par itération, les stades de l'égalisation et de la correction d'artefact, dans lequel, dans un premier passage, on égalise les premières données (B1 ) d'image initiales avec les images (S1a) de mesure de projection partielle et, dans au moins un autre passage, on égalise des données (Bn) d'image initiales corrigées avec les données (S1a) de mesure de projection partielles.

6. Procédé suivant l'une des revendications précédentes, dans lequel on effectue le stade de l'égalisation (V4) dans l'espace (RB) image.

7. Procédé suivant l'une des revendications 1 à 5, dans lequel on effectue le stade de l'égalisation (V4) dans l'espace (RP) données de projection.

8. Procédé suivant l'une des revendications précédentes, comprenant, en outre, un stade de régularisation.

9. Procédé (V) suivant l'une des revendications précédentes, dans lequel le deuxième secteur angulaire recouvre un angle de moins de 180°.

10. Procédé (V) suivant la revendication 9, dans lequel le deuxième secteur angulaire recouvre un angle dans la plage de 100° à 140°.

11. Procédé suivant l'une des revendications précédentes, comprenant, en outre, le stade d'émission (V6, V6') des premières données (BSn) d'image ou des données (Bn) d'image initiales corrigées comme image résultat.

12. Procédé suivant l'une des revendications précédentes, dans lequel, dans le stade de la correction (V5) d'artefacts, on corrige, dans les premières données (BSn) d'image, une région anatomique partielle déterminée à l'avance de l'objet (39) à examiner.

13. Dispositif (50) de reconstruction d'image ayant des moyens pour effectuer un procédé (V) suivant l'une des revendications 1 à 12, comprenant :
- une unité (51) de production pour produire (V2) des premières données (B1) d'image initiales,
- une unité (52) de sélection pour sélectionner (V3) des données (S1a) de mesure de projection partielles avec un deuxième secteur angulaire dans les données (S1) de mesure de projection, le deuxième secteur angulaire étant une partie du premier secteur angulaire,
- unité (53) d'égalisation pour égaliser (V4) les premières données (B1) d'image initiales ou des données (Bn) d'image initiales corrigées avec les données (S1a) de mesure de projection partielles des premières données (BSn) d'image étant produites et
- une unité (54) de correction d'artefact pour corriger (V5) des artefacts des premières données (BSn) d'image au moyen d'une unité (ML) de correction ayant subi un apprentissage, les données (Bn) d'image initiales corrigées étant produites.

14. Système de tomographie à ordinateur ayant un dispositif (50) de reconstruction d'image suivant la revendication 13.

15. Programme d'ordinateur ayant un code de programme, pour effectuer le procédé (V) suivant l'une des revendications 1 à 12, lorsque le programme d'ordinateur est effectué sur un ordinateur.

16. Support de données déchiffrables par ordinateur et ayant un code d'un programme d'ordinateur, pour effectuer le procédé (V) suivant l'une des revendications 1 à 12, lorsque le programme d'ordinateur est effectué sur un ordinateur.
